# EUROPÄISCHE PATENTANMELDUNG

(11) **EP 0 669 115 A1**
(43) Veröffentlichungstag der Anmeldung: **30.08.1995**
(21) Anmeldenummer: 95101584.1
(22) Anmeldetag: 07.02.1995
(51) Int. Cl.: A61F 2/30, A61F 2/36

(54) **Hüfttotalendoprothese**

(30) Priorität: 25.02.1994 DE 4406230
(71) Anmelder: FEHLING MEDICAL AG, D-63791 Karlstein (DE)
(72) Erfinder: Küsswetter, Wolfgang, Prof. Dr., D-72076 Tübingen (DE)
(74) Vertreter: Patentanwälte Westphal, Buchner, Mussgnug Neunert, Göhring

(57) **Zusammenfassung**

Der Schaft (18) einer Hüfttotalendoprothese wird individuell dem computer-tomographisch ermittelten Querschnitt des Markhohlraumes des Femurs (10) angepaßt. Der Schaft (18) hat im Querschnitt die Form einer flachen Platte, die in der Antetorsions- Ebene in das Femur (10) eingesetzt wird. Die Anpassung erfolgt zweidimensional an dem medialen und dem lateralen Rand des Schaftes (18).

## Beschreibung

Die Erfindung betrifft eine Hüfttotalendoprothese gemäß dem Oberbegriff des Anspruchs 1.

Bei Hüfttotalendoprothesen besteht das Hauptproblem in der dauerhaften Verankerung der Prothese mit ihrem Schaft in dem Femur. Bei den herkömmlichen konfektionierten zementfreien Endoprothesen kann man im wesentlichen zwei Typen unterscheiden:
Typ 1: Prothesen mit einem geraden Schaft, der rechteckigen Querschnitt aufweist;
Typ 2: Prothesen mit einem längsgekrümmten Schaft, der einen den Markraum füllenden Querschnitt aufweist.

Bei beiden Typen treten häufig ähnliche Probleme auf. Operationstechnische Schwierigkeiten entstehen durch die mangelnde Passung des Schaftes. Es können Sprengungen des Femurschaftes auftreten, der tragende Knochen kann geschädigt werden oder der Passitz der Prothese ist unzureichend. Langfristige Schwierigkeiten ergeben sich, da die biomechanischen Anforderungen, z.B. Anlage und Größe der Kontaktflächen für die Kraftübertragung von der Prothese auf den Knochen, ungenügend erfüllt sind. Die Anpassungsmöglichkeiten für die Positionierung des Gelenkkopfes sind mangelhaft, woraus sich unphysiologische Kraftflüsse in den Knochen ergeben können. Daraus resultieren langfristige Komplikationen, wie Knochenumbau, Schmerzen und Lockerungen der Prothese.

Bei einer aus der EP 0 093 869 A1 bekannten Hüfttotalendoprothese des zweiten Typs erfolgt eine Optimierung der individuellen Anpassung an den Knochenhohlraum des Femurs. Hierzu wird die Querschnittskontur des Knochenhohlraums in axial aufeinanderfolgenden Abschnitten vorzugsweise mittels Computer-Tomographie ermittelt. Der Schaft der Prothese wird in entsprechend aufeinanderfolgenden Abschnitten gemäß den so ermittelten Querschnittskonturen gestaltet. Die individuelle dreidimensionale Anpassung des Schaftes an dem Knochenhohlraum ermöglicht ein Einsetzen der Endoprothese mit einer minimalen Schädigung der Knochensubstanz.

Die individuelle dreidimensionale Anpassung des Schaftes ist allerdings ein technisch äußerst aufwendiges Verfahren, was zu hohen Kosten der Hüfttotalendoprothese führt.

Die Prothesen des Typs 1 weisen dagegen eine relativ einfache Geometrie auf, die gegenüber dem Typ 2 Vorteile bezüglich der Herstellung und des Operationsverfahrens mit sich bringt.

Der Erfindung liegt die Aufgabe zugrunde, eine Hüfttotalendoprothese des ersten Typs zu optimieren, um die oben genannten Nachteile aufgrund der mangelnden Passung zu vermeiden, ohne auf die kostengünstige Herstellung zu verzichten.

Diese Aufgabe wird erfindungsgemäß gelöst durch eine Hüfttotalendoprothese mit den Merkmalen des Anspruchs 1.

Vorteilhafte Ausführungsformen der Erfindung sind in den Unteransprüchen angegeben.

Der wesentliche Gedanke der Erfindung besteht darin, bei einer geradschäftigen Prothese mit rechteckigem Schaftquerschnitt den Schaft individuell in der Antetorsionsebene medial und lateral der Kontur des Markkanals des Femurs anzupassen. Die Antetorsionsebene kann auf der Basis bekannter bildgebender Verfahren optimal bestimmt werden.

Für die Herstellung der Hüfttotalendoprothese ergibt sich der große Vorteil, daß der Schaft aus einem vorgefertigten plattenförmigen Rohling gearbeitet werden kann, der vorzugsweise nur an seinem medialen und seinem lateralen Rand entsprechend den ermittelten Querschnitten des Markkanals z.B. mittels einer NC-gesteuerten Fräsmaschine bearbeitet werden muß.

Die erfindungsgemäße zweidimensionale Anpassung des Schaftes hat bedingt durch die optimalen präoperativen Planungsmöglichkeiten auch vorteilhafte Implantationseigenschaften zur Folge. Die wesentliche Belastung der Verankerung des Schaftes in dem Femur verläuft in der Antetorsionsebene. In dieser Ebene ist der Schaft individuell dem Markkanal des Femurs angepaßt, so daß die Hauptbelastungen optimal abgestützt werden. Aufgrund der plattenförmigen Ausbildung des Schaftes sitzt dieser mit seinem medialen Rand und seinem lateralen Rand dicht an der Compacta auf. Die Kanten des Schaftes werden dabei so konstruiert, daß sie sich leicht in die Compacta eindrücken. Dieser Effekt ist bei dem rechteckigen Querschnitt des Schaftes besonders ausgeprägt. Dadurch ergibt sich eine hohe Verdrehsicherheit des Schaftes im Markkanal des Femurs.

Eine zusätzliche Sicherung gegen Rotation des Schaftes wird in einem bevorzugten Ausführungsbeispiel dadurch erreicht, daß die den Schaft bildende Platte in ihrem proximalen Bereich auf der ventralen Seite in Längsrichtung verlaufende Rippen aufweist. Diese Rippen dienen der Komprimierung der Spongiosa des proximalen Markhohlraums und bieten eine zusätzliche Abstützung. Da die Rippen nicht zur Anlage an der Compacta kommen, ist eine individuelle Anpassung der Rippen nicht notwendig. Die Rippen können bereits an dem Rohling des Schaftes angeformt sein.

Im folgenden wird die Erfindung anhand eines in der Zeichnung dargestellen Ausführungsbeispiels näher erläutert. Es zeigen:
Figur 1 die Definition der Antetorsionsebene des Femurs,
Figur 2 einen Axialschnitt des Femurs mit eingesetzter Hüfttotalendoprothese,
Figur 3 einen Querschnitt längs der Linie A-A in Figur 2,
Figur 4 einen Querschnitt längs der Linie B-B in Figur 2,
Figur 5 einen Querschnitt längs der Linie C-C in Figur 2,
Figur 6 eine Draufsicht auf die Flachseite eines Rohlings zur Herstellung der Hüfttotalendoprothese und
Figur 7 eine Draufsicht auf die Schmalkante des Rohlings.

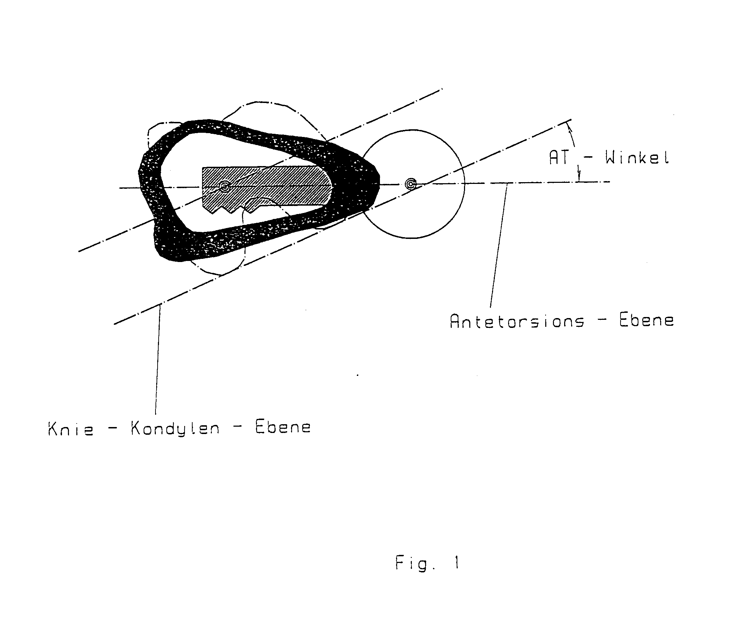

In Figur 2 ist das proximale Ende eines Oberschenkelknochens 10 in einem Axialschnitt in der Antetorsionsebene dargestellt, d.h. in der um den AT-Winkel gegen die Kondylenebene gedrehten Ebene der Schenkelhalsachse (Fig. 1). Der Markhohlraum des Femurs 10 wird mittels 3D-Rekonstruktion auf der Basis bildgebender Verfahren vorzugsweise mittels computer-tomographischer Röntgenaufnahmen in mehreren Querschnittsebenen ermittelt. Es sind Aufnahmen in etwa 10 bis 25 Ebenen erforderlich. Dabei wird im proximalen Kopfbereich des Femurs, in dem sich der Querschnitt des Markhohlraums stärker ändert, ein geringerer axialer Abstand der Aufnahmeebenen gewählt, als im Schaftbereich des Femurs 10, in welchem sich der Querschnitt des Markkanals weniger stark ändert.

Zur Herstellung der Hüfttotalendoprothese wird ein in den Figuren 6 und 7 dargestellter Rohling 12 verwendet, der in einigen wenigen Standardgrößen serienmäßig hergestellt wird. Der Rohling 12 hat die Form einer flachen Platte mit im wesentlichen rechteckigen Querschnitt. Die anteroposteriore Dikke des Rohlings 12 ist geringer als der Durchmesser des Markhohlraumes des Femurs 10 in anteroposteriorer Richtung. Im proximalen Bereich beträgt die Dicke des Rohlings 12 etwa ein Drittel oder weniger des anteroposterioren Durchmessers des Markhohlraums. Entsprechend dem sich verringernden Durchmessers des Markhohlraums in distaler Richtung beträgt die Dicke des Rohlings 12 an dessen distalem Ende etwa die Hälfte oder weniger des anteroposterioren Durchmessers des Markhohlraums. Dabei kann der Rohling 12 mit von seinem proximalen Ende gegen sein distales Ende hin abnehmender Dicke hergestellt werden. Ebenso kann die Dicke bei der späteren Bearbeitung des Rohling 12 gegen das distale Ende hin verringert werden.

Im proximalen Bereich weist der Rohling auf seiner ventralen Flachseite erhabene Rippen 14 auf, die parallel zueinander in axialer Richtung verlaufen. In dem dargestellten Ausführungsbeispiel sind drei Rippen 14 mit dreieckigem Profil nebeneinander angeordnet. Die Rippen erstrecken sich von dem proximalen Ende des Rohlings 12 über etwa ein Drittel bis maximal etwa die Hälfte der axialen Länge des Rohlings 12, wobei die Höhe der Rippen 14 in distaler Richtung abnimmt, wie am besten in Figur 7 zu erkennen ist.

Die durch die 3 D-Rekonstruktion ermittelte Größe und Lage des Durchmessers des Markhohlraumes des Femurs 10 in der Antetorsions-Schnittebene der Figur 2 werden numerisch erfaßt und in die Steuerung einer Fräsmaschine eingegeben. In den Figuren 6 und 7 sind die in den Schnittebenen rekonstruktierten Innendurchmesser des Markhohlraums des Femurs 10 nach Lage und Länge als Stecken 16 auf den Rohling 12 projiziert.

Mittels der NC-gesteuerten Bearbeitungsmaschine wird aus dem Rohling 12 entsprechend den ermittelten Querschnittswerten in der Antetorsions- ebene der Schaft 18 der Endoprothese herausgearbeitet.

Ggf. kann die Kontur des Schaftes durch den Arzt auch abweichend von dem ermittelten Querschnitt des Markhohlraumes festgelegt werden. So wird beispielsweise die Außenkontur des Schaftes 18 in dessen proximal-lateralem Bereich so gewählt, daß ein Freiraum zwischen dem Schaft 18 und dem Trochanter Major des Femurs 10 bleibt, um eine Beschädigung des Trochanter Major bei der Implantation der Endoprothese zu vermeiden.

Am proximal-medialen Ende des Schaftes 18 ist ein Hals 20 angesetzt, auf dessen konisches Ende ein nicht dargestellter Gelenkkopf aufgesteckt wird. Der Hals 20 wird vorzugsweise einstückig in den Rohling 12 eingearbeitet, wie dies aus Figur 6 hervorgeht. Lage, Größe und Winkel des Halses 20 sind vorzugsweise fest vorgegeben, wobei die individuelle Lage des Kopfes durch eine horizontale oder vertikale Verschiebung des Rohlings 12 relativ zum Femur 10 ermöglicht wird.

Zum Implantieren der Hüfttotalendoprothese in das Femur 10 wird der Kopf des Femurs 10 entlang einer in Figur 2 mit 22 bezeichneten und von der Konstruktion vorgegebenen Schnittebene abgetrennt. Dann wird der Markraum mit einem eigens dafür vorgesehenen Kastenmeißel eröffnet. Der Kastenmeißel ist mit einer Winkelmeßeinrichtung ausgestattet. Lage und Winkel des Kastenmeißels auf der Schnittebene 22 werden von der Konstruktion vorgegeben. Anschließend wird das Femur 10 vorzugsweise mit einer mit der Prothese im wesentlichen formgleichen Raspel präpariert und der Schaft 18 in das Femur 10 eingesetzt.

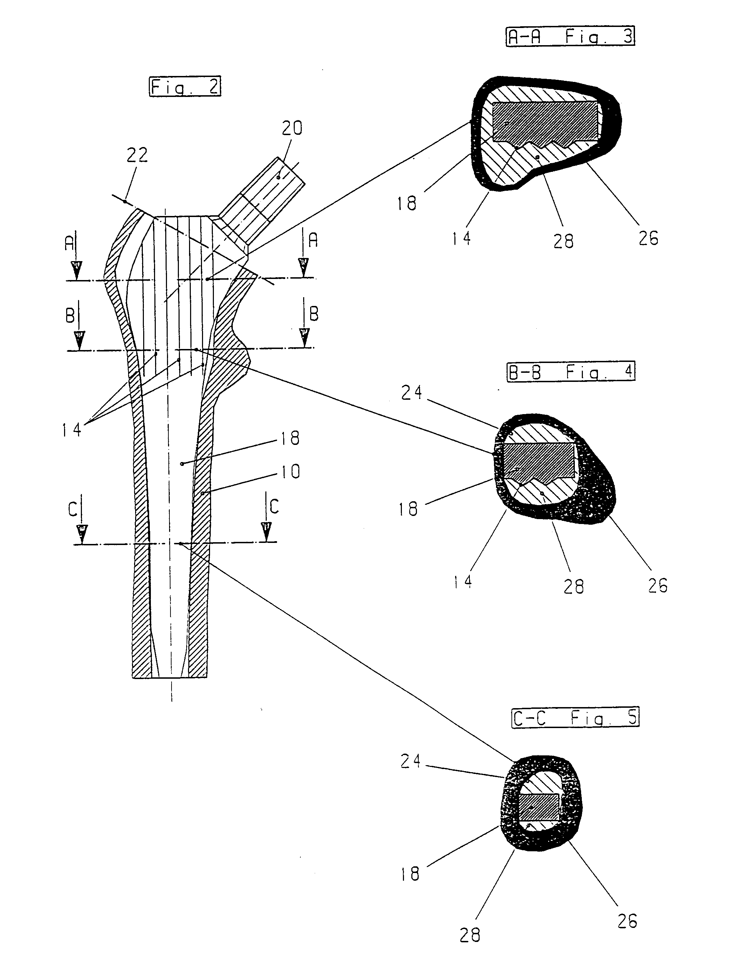

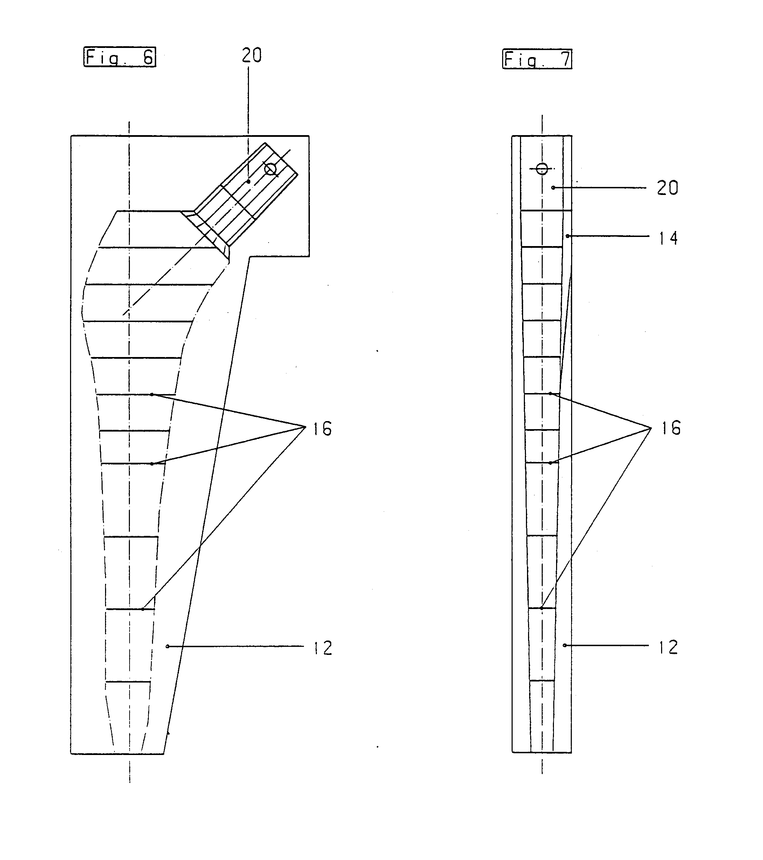

Wie aus Figur 2 und den Schnittdarstellungen der Figuren 3 bis 5 deutlich wird, sitzt dabei der Schaft 18 medial und lateral weitgehend formschlüssig in dem Markkanal 24 des Femurs 10, wobei sich aufgrund des rechteckigen Querschnittes des Schaftes 18 dessen Längskanten in die Compacta 26 des Femurs 10 eindrücken, wie dies in den Figuren 4 und 5 erkennbar ist. Aufgrund der Anpassung der medial-lateralen Breite des Schaftes 18 an den medial-lateralen Durchmesser des Markkanales 24 ergibt sich eine stabile kraftübertragende Abstützung des Schaftes 18 in dem Femur 10. Das Eindrücken der Längskanten des Schaftes 18 in die Compacta 26 ergibt zudem eine stabile Sicherung gegen eine Rotation der Endoprothese.

Im proximalen Bereich ergibt sich durch die individuelle Anpassung des Schaftes 18 an der medialen Seite eine gute Anpassung an den Adam'schen Bogen des Femurs 10, während auf der lateralen Seite ein ausreichender Abstand von dem Trochanter Major gehalten wird, wie aus den Figuren 2 und 3 erkennbar ist. Die im proximalen Bereich des Schaftes 18 ausgebildeten Rippen 14 drücken sich dabei in die Spongiosa 28 ein und bewirken im proximalen Bereich eine zusätzliche Sicherung der Endoprothese gegen Rotation im Femur 10.

## Patentansprüche

1. Hüfttotalendoprothese, mit einem Schaft, der in aufeinanderfolgenden Abschnitten dem ermittelten Querschnitt des Markhohlraumes des Femurs individuell angepaßt ist, dadurch gekennzeichnet, daß der Schaft (18) aus einer flachen Platte besteht, die im wesentlichen in der Antetorsions-Ebene in das Femur (10) einsetzbar ist und deren medialer und lateraler Rand der ermittelten Kontur des Markhohlraumes (24) angepaßt ist.

2. Hüfttotalendoprothese nach Anspruch 1, dadurch gekennzeichnet, daß die den Schaft (18) bildende Platte auf ihrer ventralen Seite in Längsrichtung verlaufende Rippen (14) aufweist.

3. Hüfttotalendoprothese nach Anspruch 2, dadurch gekennzeichnet, daß sich die Rippen (14) vom proximalen Ende des Schaftes (18) über etwa ein Drittel bis zur Hälfte der axialen Länge des Schaftes (18) in distaler Richtung erstrecken.

4. Hüfttotalendoprothese nach Anspruch 2 und 3, dadurch gekennzeichnet, daß die Rippen (14) gegen ihr distales Ende hin in der Höhe abnehmen.

5. Hüfttotalendoprothese nach einem der vorhergehenden Ansprüche, dadurch gekennzeichnet, daß der Schaft (18) einen rechteckigen Querschnitt aufweist und sich mit seinen Längskanten krafteinleitend an der Compacta (26) des Femurs (10) abstützt.

6. Verfahren zur Herstellung einer Hüfttotalendoprothese nach einem der vorhergehenden Ansprüche, dadurch gekennzeichnet, daß in axial beabstandeten vorzugsweise computer- tomographischen Schnittbildern des Femurs der Innendurchmesser des Markhohlraumes in der Antetorsions-Ebene nach Länge und Lage ermittelt wird und daß die laterale und mediale Randkontur des Schaftes entsprechend den ermittelten Querschnitten numerisch gesteuert spanabhebend aus einem Rohling in Form einer flachen Platte gearbeitet wird.

7. Verfahren nach Anspruch 6, dadurch gekennzeichnet, daß der Hals vorzugsweise standardisiert in den Rohling eingearbeitet wird und daß zur individuellen Positionierung des Gelenkkopfes relativ zum Femur der Rohling horizontal und vertikal verschoben wird.
